# EUROPÄISCHE PATENTANMELDUNG

(11) **EP 3 669 811 A1**
(43) Veröffentlichungstag der Anmeldung: **24.06.2020**
(21) Anmeldenummer: 19217370.6
(22) Anmeldetag: 18.12.2019
(51) Int. Cl.: A61B 50/31, A61F 17/00, A61B 50/30, A61B 90/00

(54) **KOMPONENTENSET ZUR MEDIZINISCHEN ERSTVERSORGEUNG**

(30) Priorität: 19.12.2018 CH 15672018
(71) Anmelder: Flawa AG, 9230 Flawil (CH)
(72) Erfinder: Härtsch, Nicolas, 9230 Flawil (CH); Steiger, Simone, 9000 St. Gallen (CH); Müller, Susanne, 9116 Wolfertswil (CH); Schwarz, Pascal, 8304 Wallisellen (CH); Oberhänsli, Reto, 8274 Tägerwilen (CH)
(74) Vertreter: IPrime Rentsch Kaelin AG

(57) **Zusammenfassung**

Die vorliegende Erfindung betrifft ein Komponenten-Set zur medizinischen Erstversorgung eines Patienten. Das Komponenten-Set umfasst einen verschliessbaren Behälter mit einer ersten Behälterwand (2), die mindestens ein erstes Innenvolumen (1) definiert. Das Komponenten-Set umfasst weiter eine Mehrzahl an zur Anordnung im ersten Innenvolumen vorgesehenen Modulen (11.2, 11.2, ...). Die Module (11.1, 11.2, ...) sind zur Behandlung und/oder Pflege eines Patienten ausgebildet. Das Komponenten-Set umfasst weiter ein Sensorelement zur Detektion eines geöffneten Zustands des verschliessbaren Behälters und eine Sendeeinheit zur Kommunikation mit einem Rechner. Eine Steuereinheit verarbeitet die Detektion eines geöffneten Zustands des Behälters und erzeugt ein entsprechendes Signal.

Die Erfindung umfasst weiter ein System zur Versorgung eines Gebäudes und/oder Gebäudekomplexes mit Notfallausrüstung zur medizinischen Erstversorgung und ein Verfahren zum Betreiben eines solchen.

## Beschreibung

Die vorliegende Erfindung betrifft Vorrichtungen und Verfahren zur medizinischen Erstversorgung eines Patienten in der Form von Komponenten-Sets, Systemen und Verfahren zur medizinischen Erstversorgung eines Patienten gemäss Oberbegriffen der unabhängigen Ansprüche.

### Technologischer Hintergrund

In Notfallsituationen kann die medizinische Erstversorgung entscheidend sein, um bei einem Unfallopfer bleibende Schäden zu vermeiden. Es ist daher üblich, dass in Unternehmen oder anderweitig mehr oder weniger öffentlich zugänglichen Räumen "Notfallkoffer" an strategisch günstigen Stellen platziert werden, um im Bedarfsfall zur Erstversorgung eines Patienten zur Verfügung zu stehen. Obschon mit der Zurverfügungstellung eines Notfall-Sets ein erster Schritt in die richtige Richtung unternommen wurde, besteht nach wie vor das Risiko, dass ein solches, öffentlich zugängliches Set unter Umständen nicht vollständig und/oder nicht in einwandfreiem Zustand ist. So kann es durchaus vorkommen, dass für einen Fabrikationsraum, der mit einer Reihe von Notfall-Sets ausgestattet ist, dieser nur mit einem beträchtlichen Aufwand an regelmässiger Wartung und einer durchorganisierten Verantwortlichkeit für die Notfall-Sets eine Aufrechterhaltung der unbedingten Zugänglichkeit für den Notfall ermöglichen kann. In einigen Fällen hat zudem ein Diebstahl an Material dazu geführt, dass entscheidende Materialien zur Versorgung eines Patienten im Notfall nicht verfügbar waren. Aus diesem Grund kann es vorkommen, dass einige Betreiber von öffentlich zugänglichen Bereichen oder von Firmenräumen ganz auf die Bereitstellung eines Notfall-Sets verzichten, mit entsprechenden versicherungstechnischen Folgen.

Eine Möglichkeit, Diebstahl von Material zu erschweren, wäre es, die Notfall-Sets analog zu Feuerlöschern und Löschdecken mit versiegelten Verschlüssen zu versehen, sodass ein Herumbasteln am Verschluss bei einem Kontrollgang mindestens entdeckt würde. Dies setzt allerdings nach wie vor voraus, dass entsprechende Kontrollgänge geplant und organisiert sind, sowie dass die entsprechenden Siegel im Bedarfsfall, also bei Vorliegen eines konkreten Notfalls, ohne Weiteres aufgebrochen werden können, um den Inhalt des Notfallkoffers zugänglich zu machen. Es stellt sich auch die Frage, was mit einem angebrochenen Notfallkoffer zu unternehmen wäre. Entsprechend müssten nach einer Verwendung die benutzten Materialien notiert, ersetzt und von einem Fachmann vorzugsweise in den Koffer zurückbefördert werden, sowie zusätzlich das Siegel erneuert werden. Für viele Firmen ist dieser Aufwand zu hoch, sodass ein angebrochener Koffer als kontaminiertes Material bereits vollständig ersetzt wird, was allerdings zu erhöhtem Materialverbrauch und vergleichsweise höheren Kosten führt.

Es besteht somit ein Bedarf an Lösungen, die es erlauben, in einem mehr oder weniger öffentlich zugänglichen Raum, wie z.B. einem öffentlichen Gebäude oder einem Firmengelände, ein Notfallsystem bereitzustellen, welches effizient im Unterhalt ist und garantiert, dass in einer Notfallsituation die entsprechenden Materialien zur Erstversorgung eines Patienten vorhanden sind.

### Darstellung der Erfindung

Es ist somit eine Aufgabe der vorliegenden Erfindung, ein Komponenten-Set, ein System und ein Verfahren bereitzustellen, welche mindestens einen Nachteil des Bekannten beheben. Insbesondere sollen ein Komponenten-Set, ein System und ein Verfahren bereitgestellt werden, welche einen effizienten Unterhalt an Mitteln zur Erstversorgung eines Patienten in einem öffentlichen und/oder betriebsinternen Raum ermöglichen. Dieses System ist vorzugsweise zuverlässig und ermöglicht es, die Kosten im Unterhalt des Notfallsystems zu senken, sowie eine medizinische Erstversorgung in stetiger Qualität zu gewährleisten.

Diese Aufgabe wird mit einem Komponenten-Set, einem System und einem Verfahren gemäss kennzeichnendem Teil der unabhängigen Ansprüche gelöst.

Ein Aspekt der vorliegenden Erfindung betrifft ein Komponenten-Set zur medizinischen Erstversorgung eines Patienten. Das Komponenten-Set umfasst einen verschliessbaren Behälter mit einer ersten Behälterwand, die mindestens ein erstes Innenvolumen definiert. Das Komponenten-Set umfasst weiter eine Mehrzahl an zur Anordnung im ersten Innenvolumen ausgebildeten Modulen. Dabei sind die Module zur Behandlung und/oder Pflege eines Patienten ausgebildet. Bevorzugt sind die Module zur Behandlung und/oder Pflege eines Patienten ausgebildet, der unter einem spezifischen Zustand leidet. Das erfindungsgemässe Komponenten-Set umfasst weiter ein Sensorelement zur Detektion eines geöffneten und/oder geschlossenen Zustandes des verschliessbaren Behälters. Weiter umfasst es eine Sendeeinheit zur Kommunikation mit einem Rechner, und eine Steuereinheit zur Verarbeitung der Detektion des Zustands des Behälters und zur Erzeugung eines Signals.

Im Sinne der vorliegenden Erfindung kann ein verschliessbarer Behälter beispielsweise als ein Körper verstanden werden, der in seinem Inneren einen im Wesentlichen gegen einen Aussenraum abgrenzbaren Hohlraum aufweist. Dieser Hohlraum kann z.B. mindestens einen Teil des ersten Innenvolumens definieren. Unter verschliessbar kann z.B. verstanden werden, dass der Behälter sich mittels einer Behälterkomponente in einen Zustand überführen lässt, der keinen Zugriff von aussen auf das Innenvolumen ermöglicht. In einer besonderen Ausführungsform kann dies gewährleistet werden mit einer ersten Behälterwand, die mindestens ein erstes Volumen definiert, und einem entsprechenden Deckel, der das mindestens erste Volumen derart abschliesst, dass dieses im verschlossenen Zustand von aussen nicht zugängig ist.

In einer besonderen Ausführungsform umfasst das Sensorelement einen berührungslos schaltenden Sensor, der ein Magnetfeld erkennt, insbesondere einen berührungslos schaltenden Sensor der zwei ferromagnetischen Schaltzungen aufweist. Besonders bevorzugt umfasst das Sensorelement einen Reed-Schalter umfassend zwei ferromagnetische Schaltzungen.

In einer besonderen Ausführungsform ist die Behälterwand einstückig ausgebildet. Dies kann z.B. in der Form eines spritzgegossenen Formteils erreicht werden, das ein erstes Innenvolumen definiert.

In einer besonderen Ausführungsform kann der Behälter mit entsprechenden Elementen zum Verschliessen des Behälters ausgestattet sein. Geeignete Elemente zum Verschliessen eines Behälters sind Verschlüsse, Rastnasen, Klettverschlüsse, Klebebänder etc.

Im Sinne der vorliegenden Erfindung kann unter Modulen, die zur Behandlung und/oder Pflege eines Patienten ausgebildet sind, verstanden werden, dass die entsprechenden Module eine konfektionierte Auswahl an Behandlungs- und/oder Pflegegegenständen umfassen, die zur konkreten Behandlung, insbesondere Erstversorgung eines spezifischen Zustands zusammengestellt wurden. Die Module können zum Beispiel als Schachteln, Packungen oder Boxen ausgestaltet sein, deren Inhalt die entsprechende Auswahl an Behandlungsgegenständen umfasst. Alternativ oder ergänzend können Module als Beutel, Taschen und/oder Tüten ausgestaltet sein, erneut mit einer entsprechenden Auswahl an Behandlungsgegenständen. Die Module können in einer bevorzugten Ausführungsform farblich oder strukturell gekennzeichnet sein, sodass sie rasch einem entsprechenden Verwendungszweck zur Behandlung eines spezifischen Zustands zugeführt werden können. Im Sinne der vorliegenden Erfindung kann auch ein einzelner Pflege- und/oder Behandlungsgegenstand als Modul angesehen werden. So kann zum Beispiel eine Schere als ein bestimmtes Modul im Komponenten-Set betrachtet werden. Ein solches aus einem Einzelgegenstand bestehendes Modul kann eine Verpackung aufweisen, oder unverpackt im Komponenten-Set angeordnet sein.

In einer besonderen Ausführungsform sind die Module zur einmaligen, fallbezogenen Behandlung und/oder Pflege eines Patienten ausgebildet. Die Einmaligkeit kann zum Beispiel durch ein entsprechendes Verpackungssiegel gewährleistet werden.

In einer besonders bevorzugten Ausführungsform sind die Module zusätzlich jeweils mit einer Anleitung versehen, die einen entsprechenden Ablauf der medizinischen Erstversorgung für den spezifischen Zustand beschreibt.

Im Sinne der vorliegenden Erfindung kann unter einem spezifischen Zustand, unter dem ein Patient leidet, eine Verletzung oder eine anderweitige gesundheitliche Beeinträchtigung verstanden werden. Die Module der vorliegenden Erfindung können besonders bevorzugt eingesetzt werden für Quetschungen, Stauchungen, Prellungen, Schnittwunden, stumpfes und/oder spitzes Trauma, Knochenbrüche, Amputationen, Verbrennungen, Verätzungen, Unterkühlungen, Vergiftungen und/oder Schockzustände. Entsprechend können Module Konfektionierungen umfassen, die Wundverbände, Druckverbände, Kompressen zur Wundversorgung, Kalt-/Warmkompressen, Hyperventilationsmasken, Splitterpinzetten, Rettungsdecken, Skalpelle, Handschuhe, Desinfektionsmittel etc. enthalten. Die Module können alternativ oder zusätzlich ausgebildet sein, um eine Verletzung eines bestimmten Körperteils zu behandeln. So kann z.B. ein Modul vorgesehen sein, um konkret Verletzungen der Finger in einer medizinischen Erstversorgung zu behandeln. Ein solches Set kann z.B. Fingerverbände, Fingerspitzenverbände, Pflaster und Bandagen verschiedener Grössen sowie Wundreinigungstücher umfassen. Für gewisse spezifische Zustände kann es auch angebracht sein, eine Mehrzahl an Modulen zu kombinieren. Diese Module können entsprechend gekennzeichnet werden. So kann z.B. in einer erfindungsgemässen Ausgestaltung ein Modul mit Desinfektionsmitteln vorgesehen sein, welches sich zur Kombination mit einem beliebigen anderen Modul, welches Verbandsmaterial enthält, eignet. Als weiteres Modul kann z.B. ein Set mit sterilen Handschuhen vorgesehen sein, welches beliebig bei jeder Art von Behandlung eines spezifischen Zustands zum Einsatz kommen kann.

In einer bevorzugten Ausführungsform ist die erste Behälterwand des verschliessbaren Behälters so ausgestaltet, dass die Mehrzahl an zur Anordnung im ersten Innenvolumen vorgesehenen Modulen in dieser effizient und übersichtlich verstaut werden kann. Dies kann z.B. damit gewährleistet werden, dass jedem Modul eine entsprechend ihrer Grösse und Form gekennzeichnete Zone zugeteilt wird, in der das Modul verstaut werden kann.

Durch das Komponenten-Set der vorliegenden Erfindung ist es möglich, z.B. die Benutzung eines entsprechenden Komponenten-Sets zur medizinischen Erstversorgung zu erkennen. Das Komponenten-Set kann durch die Sendeeinheit jeweils mitteilen, wenn das Komponenten-Set geöffnet und/oder geschlossen wurde. Dies kann betriebsintern z.B. einen Prozess auslösen, bei dem die entsprechende Überprüfung des Komponenten-Sets in die Wege geleitet wird. Allfällig fehlendes Material kann somit rasch ersetzt werden. Weiter kann durch das Erkennen eines geöffneten und/oder geschlossenen Zustands eine solche Übertragung von Informationen sehr energieeffizient stattfinden. Das Komponenten-Set kann in diesem Beispiel so ausgelegt werden, dass es energetisch in einem abgeschalteten Ruhezustand verweilt, bis das Komponenten-Set geöffnet wird. Wird ein solches Öffnen registriert, so kann erst der elektronische Prozess beginnen, bei dem die entsprechende Öffnung von der Sendeeinheit übermittelt wird. Dies gewährleistet, dass entsprechend in einem Gebäude und/oder Gebäudekomplex verteilt angebrachte Komponenten-Sets eine lange Standzeit aufweisen, und Verlass darauf besteht, dass während eines Einsatzes die entsprechende Öffnung des Komponenten-Sets erkannt wird. Diese Komponenten-Sets zehren nicht dauernd Energie und sind entsprechend energieeffizient und mit langen Standzeiten einsetzbar. Selbstverständlich kann ein analoges Vorgehen bei Erkennen des Schliessens eines Behälters ablaufen.

In einer besonderen Ausführungsform ist die erste Behälterwand einstückig ausgebildet.

In einer alternativen Ausführungsform ist die erste Behälterwand mehrstückig ausgebildet. Dies kann z.B. bedeuten, dass eine Aussenschale aus einem ersten Stück besteht und eine Innenverkleidung aus einem zweiten Stück. Diese Innenverkleidung kann so ausgestaltet sein, dass sie das mindestens ein erstes Innenvolumen in kleinere Bereiche unterordnet. Selbstverständlich kann auch in der einstückigen Ausführungsform das Innenvolumen durch entsprechende an der Innenfläche der ersten Behälterwand ausgebildete Strukturen in Unterbereiche untergeordnet werden.

Für die Lehre der vorliegenden Erfindung können verschiedene Sensorelemente zur Detektion eines geöffneten und/oder geschlossenen Zustands des verschliessbaren Behälters verwendet werden.

In einer besonderen Ausführungsform umfasst der verschlossene Behälter einen Stromkreis, der im geöffneten Zustand unterbrochen oder erst ermöglicht wird. Dies kann z.B. über Kontakte an Verschlusselementen, welche dem Verschliessen des verschliessbaren Behälters dienen, ermöglicht werden.

In einer besonderen Ausführungsform umfasst das Komponenten-Set ein zweites Sensorelement zur Detektion eines bestimmten Moduls aus der Mehrzahl an zur Anordnung im ersten Innenvolumen vorgesehenen Modulen. Dieses zweite Sensorelement kann zum Beispiel ein optisches Sensorelement sein.

In einer besonderen Ausführungsform ist dieses zweite Sensorelement ausgelegt, um elektromagnetisch die Anwesenheit eines bestimmten einer Mehrzahl an zur Anordnung im ersten Volumen vorgesehenen Modulen innerhalb der ersten Behälterwand zu erkennen. So kann in einer besonders bevorzugten Ausführungsform das Komponenten-Set so ausgestaltet sein, dass es die Anwesenheit eines neuen Moduls erkennt und entsprechend das Vorhandensein des neuen Moduls protokolliert und/oder übermittelt.

In einer besonderen Ausführungsform ist das zweite Sensorelement ein Lesegerät für Transponder zur Identifizierung eines Moduls aus der Mehrzahl an zur Anordnung im ersten Innenvolumen vorgesehenen Modulen zur Behandlung und/oder Pflege eines Patienten.

Im Sinne der vorliegenden Erfindung kann die Sendeeinheit zur Kommunikation mit einem Rechner als Netzwerkanschluss ausgestaltet sein.

In einer besonderen Ausführungsform umfasst die Sendeeinheit einen LAN-Anschluss, insbesondere eine WLAN-Antenne.

In einer weiteren besonderen Ausführungsform umfasst die Sendeeinheit Mittel zur Übertragung im Nahfeldbereich, insbesondere einen NFC- und/oder Bluetooth-Sender.

In einer besonderen Ausführungsform ist die Sendeeinheit ausgestaltet, um mit einem Long Range Wide Area Network Informationen zu übertragen.

Im Sinne der vorliegenden Erfindung kann als Steuereinheit ein Prozessor verwendet werden.

In einer besonderen Ausführungsform umfasst die erste Behälterwand definierte Aufnahmebereiche zur Aufnahme mindestens eines Moduls der Mehrzahl an Modulen. Bevorzugt weist die erste Behälterwand definierte Aufnahmebereiche auf, die zur Aufnahme eines definierten Typs eines Moduls der Mehrzahl an Modulen ausgebildet sind. Diese Aufnahmebereiche können z.B. mit entsprechenden Farbkennzeichnungen, über die Form oder über entsprechend ausgebildete Strukturen so ausgelegt sein, dass stets ein bestimmtes Modul der Mehrzahl an Modulen, oder ein bestimmter Modultyp der Mehrzahl an Modulen für diesen bestimmten Bereich geeignet ist. Die definierten Aufnahmebereiche können auch als Kammern ausgebildet sein oder als separat verschliessbare Fächer.

In einer besonderen Ausführungsform umfasst die erste Behälterwand Magnete, welche ausgelegt sind, um mit entsprechenden Gegenmagneten der Module in Wirkverbindung zu treten, sodass die Module innerhalb des Behälters an Ort und Stelle gehalten werden.

In einer besonderen Ausführungsform umfasst die Behälterwand Mittel, um elektromagnetisch das Vorhandensein eines bestimmten Moduls zu erkennen.

In einer besonderen Ausführungsform kann dies elektromagnetische Erkennen über die Positionierung von Magneten und das Erkennen eines elektromagnetischen Feldes beim Vorhandensein der Module bewerkstelligt werden. Alternativ oder ergänzend kann eine RFID-Antenne oder eine Mehrzahl an RFID-Antennen entlang der Behälterwand so angeordnet sein, dass ein entsprechend im Nahfeld der Antenne detektierbares Modul erkannt wird.

In einer besonderen Ausführungsform umfasst das Komponenten-Set ein zweites Sensorelement, zur Detektion einer Abwesenheit eines bestimmten Moduls aus der Mehrzahl an zur Anordnung im ersten Innenvolumen ausgebildeten Modulen. Dies kann z.B. eine optische, physikalische, elektromagnetische Detektion sein.

In einer besonderen Ausführungsform kann ein Piezo-Sensor ausgelegt sein, um auf physikalische Weise das Vorhandensein eines Moduls zu detektieren.

In einer alternativen oder ergänzenden Ausführungsform kann das zweite Sensorelement eine RFID-Antenne umfassen, die ausgelegt ist, um einen entsprechenden Transponder an einem Modul zu erkennen. Ebenso denkbar ist eine Leseeinheit, die auf optischer Erkennung basiert und einzelne optische Faktoren an den Modulen jeweils erkennt. Die optische Erkennung kann auch einfach darauf beruhen, dass ein entsprechender Bereich der Behälterwand als leer erkannt wird. Dies kann mit einer einfachen Lichtschranke bewerkstelligt werden, z.B.

In einer besonderen Ausführungsform umfasst das Komponenten-Set eine Empfangseinheit zum Empfang von Daten. Bevorzugt ist die Empfangseinheit ein Bestandteil der Sendeeinheit.

Dadurch kann das Komponenten-Set nicht nur Zustandsinformationen versenden, sondern kann auch mit einem Netzwerk interagieren. Das Komponenten-Set kann z.B. Informationen zu den jeweils bestückten Modulen empfangen. Ebenso kann das Komponentensetz Anweisungen empfangen, z.B. die Anweisung eine Bestandesaufnahme durchzuführen, in dem mit dem zweiten Sensorelement die Anwesenheit/Abwesenheit eines bestimmten Moduls detektiert wird.

In einer besonderen Ausführungsform umfasst das Komponenten-Set eine Quelle für elektrische Energie.

In einer weiteren besonderen Ausführungsform ist diese Quelle für elektrische Energie eine aufladbare Batterie. Die aufladbare Batterie kann in die erste Behälterwand eingelassen sein, sodass lediglich ein von aussen zugänglicher Stromanschluss erkennbar bleibt. Alternativ oder ergänzend wäre es auch denkbar, die Quelle für elektrische Energie als induktiv aufladbare Batterie komplett von der ersten Behälterwand umschlossen auszubilden. Dadurch wäre ein kabelloses Ladesystem als Quelle für elektrische Energie des Komponenten-Sets möglich.

In einer weiteren besonderen Ausführungsform ist die Energiequelle so ausgebildet, dass sie elektrische Energie erst dann zur Verfügung stellt, wenn der verschliessbare Behälter in einem geöffneten Zustand ist.

Zusätzlich kann das Komponenten-Set mit einem oder mehreren Spulen zur drahtlosen Energieübertragung ausgestaltet sein, welche dazu dienen, RFID-Transponder zu aktivieren.

In einer besonderen Ausführungsform umfassen die Module passive RFID-Tags.

In einer besonderen Ausführungsform umfasst das Komponenten-Set mindestens ein Identifikationsmittel zur Identifikation eines bestimmten Moduls der Mehrzahl an Modulen.

In einer besonders bevorzugten Ausführungsform umfasst das Komponenten-Set ein eigenes Identifikationsmittel für jedes Modul der Mehrzahl an Modulen.

In einer besonderen Ausführungsform umfasst das Komponenten-Set eine zweite Behälterwand. Diese zweite Behälterwand kann als Deckel für die erste Behälterwand ausgestaltet sein.

In einer besonders bevorzugten Ausführungsform ist die zweite Behälterwand so ausgestaltet, dass sie mindestens ein zweites Innenvolumen definiert. Dieses zweite Innenvolumen kann z.B. so ausgebildet sein, dass das zweite Innenvolumen ebenso in Bereiche unterteilt ist, die jeweils zur Aufnahme eines Moduls aus der Mehrzahl an Modulen ausgebildet sind. Die zweite Behälterwand kann ebenso mit Sensoren und Identifikationsmitteln ausgestattet sein, welche es ermöglichen, ein Modul in seinem bestimmten Bereich zu erkennen und die Anwesenheit eines Moduls an die Steuereinheit und/oder Sendeeinheit zu übertragen.

In einer besonders bevorzugten Ausführungsform sind die erste und die zweite Behälterwand als komplementäre Schalen eines Koffers ausgebildet. In diesem Fall wäre das Kofferinnenvolumen die Summe aus dem ersten Innenvolumen und dem zweiten Innenvolumen. Der Verschluss kann z.B. als Schnappverschluss oder Spannverschluss ausgebildet sein, wie er in handelsüblichen Koffern vorkommt. Zusätzlich kann dieser Schnapp- oder Spannverschluss mit einem Kippschalter oder einem elektrischen Kontakt ausgestaltet sein, sodass ein geöffneter Zustand des Koffers detektiert wird. Alternativ kann selbstverständlich auch der geschlossene Zustand detektiert werden, was dazu führt, dass auch der offene Zustand erkannt würde.

In einer besonderen Ausführungsform umfasst die erste Behälterwand und/oder die zweite Behälterwand mindestens eine Antenne, sodass ein elektromagnetisches Signal zur Detektion eines Moduls in ihrem Wirkbereich empfangbar ist. Besonders bevorzugt ist die Antenne so ausgebildet, dass ein elektromagnetisches Signal zur Detektion eines Moduls in ihrem Wirkbereich im Nahfeld empfangbar ist.

In einer besonderen Ausführungsform sind einzelne Bereiche zueinander dergestalt isoliert, dass ein Signal eines Identifikationsmittels innerhalb eines Bereichs nicht von einem Sensor und/oder einer Antenne erkannt würde, die zur Detektion eines Identifikationsmittels in einem benachbarten Bereich ausgerichtet ist. Dies kann insbesondere dadurch erreicht werden, dass die entsprechenden Antennen zur Detektion eines Identifikationsmittels an einem Modul z.B. gerichtet sind auf die Ausrichtung des Identifikationsmittels bei korrekter Platzierung in dem definierten Bereich der Behälterwand.

In einer besonderen Ausführungsform umfassen die Module des Komponenten-Sets einen Manipulationsschutz. Dieser Manipulationsschutz kann am Modul so ausgestaltet sein, dass bei einem Manipulieren das Identifikationsmittel des Moduls nicht mehr einwandfrei funktioniert. Dadurch kann auch ein benutztes Modul, oder ein anderweitig kontaminiertes Modul, welches wieder in den richtigen Bereich der Behälterwand des Komponenten-Sets versorgt wurde, als fehlend erkannt werden. Dadurch kann z.B. ein Prozess zum Ersetzen des Moduls in Gang gesetzt werden.

In einer besonderen Ausführungsform umfasst das Identifikationsmittel des Moduls Informationen hinsichtlich der Art des Moduls. Besonders bevorzugt umfasst das Identifikationsmittel zudem Informationen zum Herstellungs- und/oder Konfektionierungsdatum des Moduls.

In einer besonders bevorzugten Ausführungsform umfasst das Identifikationsmittel zudem Ablaufdaten mindestens einer Komponente eines bestimmten Moduls. In einem konkreten Beispiel kann ein Modul zur Behandlung von Quetschungen neben Kompressen, Verbandmaterial und Dreieckstüchern eine kühlende Salbe enthalten. Das Identifikationsmittel kann ausgelegt sein, um das früheste Ablaufdatum zu umfassen. Das Identifikationsmittel kann auch ausgelegt sein, um alle Ablaufdaten aller Komponenten des entsprechenden Moduls für Quetschungen zu umfassen. So kann analog für alle Module vorgegangen werden, sodass stets Informationen bereitstehen hinsichtlich der in einem Komponenten-Set vorhandenen Module, ihrer Ablaufdaten und der Integrität der entsprechenden Module.

In einer weiteren besonderen Ausführungsform sind das Sensorelement, die Sendeeinheit und die Steuereinheit in einem definierten Kontrollabteil ausgebildet, welches im Innenvolumen der ersten und/oder zweiten Behälterwand untergebracht ist. Die Kontrolleinheit kann in einer besonders bevorzugten Ausführungsform gegenüber dem übrigen Volumen, wo die Bereiche für die Module definiert sind, abgeschlossen sein. Zu diesem Zweck kann z.B. eine Behälterwand aus mehreren miteinander kraftschlüssig verrastbaren Formteilen bestehen, welche einen Zugang zum Kontrollabteil und der entsprechenden Elektronik verhindern. Zusätzlich kann durch die Gestaltung der Formteile eine entsprechende Führung der Antennen gewährleistet werden, sodass die einzelnen Bereiche in ihrem Nahfeld erkennbar und die Anwesenheit eines Moduls detektierbar ist, wie es oben geschildert wurde. Die Steuereinheit kann so ausgelegt sein, dass sie mit einer internen Zeiterfassung in periodischen Abständen hochgefahren wird und eine Bestandeskontrolle der einzelnen Module vornimmt. Dies kann z.B. innerhalb von gewissen Wartungsintervallen geschehen. Alternativ oder ergänzend kann eine solche Kontrolle jeweils immer dann ausgeführt werden, wenn das Komponenten-Set an einen Stromanschluss angeschlossen ist und/oder wieder angeschlossen wurde. In diesem Fall wäre die Steuereinheit so ausgebildet, dass bei einem Stromanschluss eine Bestandesaufnahme aller Module durchgeführt wird. Im Betrieb würde dies bedeuten, dass die Steuereinheit die Sensoren und/oder Antennen aktiviert, welche die Identifikationsmittel der Module erfassen sollen. Wird ein bestimmtes oder eine Mehrzahl an Modulen als fehlend erkannt, z.B. weil kein Signal von der entsprechenden Antenne an die Steuereinheit zurückgelangt, oder werden alle Module als vorhanden und integer erkannt, indem z.B. alle Sensoren und/oder Antennen das entsprechende zugeordnete Modul erkannt haben, so wird über die Sendeeinheit mit einem Rechner ein Kommunikationskanal geöffnet und die entsprechende Information übertragen. Alternativ oder ergänzend kann das Komponenten-Set mit einer Speichereinheit ausgestattet sein, welche jeweils den Befund der Kontrolle abspeichert. In diesem Fall kann z.B. die Sendeeinheit passiv zum Empfangen eines Signals ausgebildet sein und erst auf dieses Signal hin die Bestandesaufnahme, welche durch die letzten Kontrollen ermittelt wurde, an den Rechner oder den Anfragenden übersenden.

In einer besonderen Ausführungsform umfasst das Komponenten-Set ein Identifikationsmittel auf der Aussenseite, z.B. einen Transponder und/oder einen Barcode. Dieses Identifikationsmittel kann mittels eines elektrischen Lesegeräts ausgelesen werden und gibt Informationen zum Letztgenannten oder zur Gesamthistorie an protokollierten Bestandesaufnahmen. Durch eine solche Ausgestaltung kann die Wartung von Komponenten-Sets zur medizinischen Erstversorgung, welche in einem Gebäude und/oder Gebäudekomplex verteilt angebracht sind, zusätzlich erleichtert werden. Ein Kontrollgang würde das Auslesen dieses äusseren Identifikationsmittels eines Komponenten-Sets erfordern und das Lesegerät oder ein damit verbundener Rechner hätte automatisch die vollständigen Bestandesinformationen zu den vorhandenen Modulen, sowie zur Integrität der vorhandenen Module.

In einer besonderen Ausführungsform ist das Komponenten-Set mit einem Befestigungsmittel versehen, mit dem das Komponenten-Set z.B. an einer Wand abnehmbar befestigbar ist. Das Befestigungsmittel kann zudem mit der Steuereinheit wirkverbunden sein, sodass die Steuereinheit aktiviert wird, wenn das Komponenten-Set von der Wand entfernt wird. In einer alternativen Ausführungsform ist das Komponenten-Set zur festen Montage an eine Wand ausgestaltet, so dass es nicht ohne weiteres abnehmbar ist.

In einer besonders bevorzugten Ausführungsform detektiert das Komponenten-Set über das Befestigungsmittel, ob es von der Wand getrennt wurde. In diesem Fall startet die Steuereinheit des Komponenten-Sets auf und registriert, wie lange das Komponenten-Set von seiner Positionierung entfernt worden ist, wie lange und ob es geöffnet worden ist, sowie im Anschluss oder während des Gebrauchs, welches der Module aus der Mehrzahl an Modulen aus dem Komponenten-Set entfernt wurden.

Diese Informationen können wie die oben genannte Bestandesaufnahme durch eine Steuereinheit, welche ausgebildet ist, diese Informationen zu verarbeiten, über die Sendeeinheit an einen Drittrechner übertragen werden.

In einer besonderen Ausführungsform ist die Steuereinheit so ausgebildet, dass sie einen Stromunterbruch des Komponenten-Sets detektiert und hochfährt, indem sie eine interne Stromquelle verwendet, z.B. eine wiederaufladbare oder eine Einweg-Batterie. Im Betrieb würde dies bedeuten, dass ein korrekt an einer Wand platziertes Komponenten-Set mit einer Stromversorgung gekoppelt wäre. Wird das Komponenten-Set von der Wand entfernt, so wird ein Stecker z.B. aus dem Komponenten-Set entfernt und die Stromversorgung unterbrochen. Die Steuereinheit ist ausgebildet, um mit diesem Unterbruch mittels der Batterie hochzufahren und eine Bestandesaufnahme in die Wege zu leiten. Die Steuereinheit kann ausgebildet sein, die Bestandesaufnahme nach periodischen Zeitabständen aufzunehmen. Alternativ oder ergänzend kann die Steuereinheit ausgebildet sein, die Bestandesaufnahme dann auszuführen, wenn der Stromzugang wiederhergestellt ist.

In einer weiteren bevorzugten Ausführungsform umfasst das Komponenten-Set zudem ein Mittel zur Erkennung der Geo-Position des Komponenten-Sets. Dazu kann z.B. ein Live-Tracking-Gerät in einer Behälterwand verbaut werden. Dieses Live-Tracking-Gerät kann z.B. ein GPS-Gerät mit einem GSM-Modul mit SIM-Karte sein, wie sie handelsüblich in Mobiltelefonen verwendet werden.

In einer besonderen Ausführungsform umfasst das Komponenten-Set mindestens eine Tragehilfe. Diese Tragehilfe kann z.B. in der Form eines Tragegriffes oder einer Trageschleife ausgebildet sein. Mit einem erfindungsgemässen Komponenten-Set wird es möglich, eine Lösung zur medizinischen Erstversorgung in einem Gebäude und/oder Gebäudekomplex bereitzustellen, welche die Wartung und Instandhaltung eines solchen Systems massgeblich erleichtert. Die Komponenten-Sets ermöglichen eine medizinische Erstversorgung, die zielgerichtet auf die Bedürfnisse des entsprechenden Notfalls ausgerichtet sind. Zudem besteht eine ständige und stetige Qualitätskontrolle für die einzelnen Module, die zur Behandlung einer spezifischen Notfallsituation verbaut werden. Es ist zudem ohne weiteren logistischen Aufwand möglich, innerhalb eines Gebäudes und/oder Gebäudekomplexes je nach möglicher Notfallsituation andere Komponenten-Sets bereitzustellen, welche jeweils für die entsprechende Notfallsituation massgeschneiderte Module aufweisen. Die Komponenten-Sets der vorliegenden Erfindung sind ausgebildet, um vollständig autark zu funktionieren. Die Verbindung zwischen den Komponenten-Sets kann z.B. mittels eines Smartphones, wie weitum erhältlich, bereits sämtliche Kontroll- und Bestandesfunktionen ermöglichen, wie sie oben als mögliche Ausführungsformen beschrieben wurden. Das Komponenten-Set kann zudem mit weiteren Komponenten-Sets zu einem Netzwerk zusammengeschlossen werden, welches eine Reihe von Auswertungen ermöglicht hinsichtlich z.B. der Nutzung, der Verbrauchswerte und der Zugänglichkeit der einzelnen Komponenten-Sets enthalten.

Für einen Fachmann versteht es sich von selbst, dass alle oben genannten Ausführungsformen in einer Ausführung eines erfindungsgemässen Komponenten-Sets beliebig kombinierbar verwirklicht werden können, sofern sie sich nicht gegenseitig ausschliessen.

Ein weiterer Aspekt der vorliegenden Erfindung betrifft ein System zur Versorgung eines Gebäudes und/oder Gebäudekomplexes mit Notfallausrüstung zur medizinischen Erstversorgung eines Patienten. Ein Beispiel für einen solchen Gebäudekomplex wäre ein Firmengelände mit Fabrikationshallen, Betrieben, Lager etc. Das System umfasst eine Mehrzahl an Komponenten-Sets. Bevorzugt umfasst das System eine Mehrzahl an Komponenten-Sets, wie sie oben geschildert wurden. Die Module können besonders bevorzugt an die entsprechenden Bedürfnisse des Gebäudes und/oder Gebäudekomplexes angepasst werden. So können Module, die im Bereich einer chemischen Fabrikation in Komponenten-Sets eingesetzt werden, eher auf die Behandlung chemischer Notfallsituationen, wie z.B. Verätzungen und/oder Verbrennungen und/oder Vergiftungen, ausgelegt sein. Jedes Komponenten-Set des Systems umfasst eine Mehrzahl an Modulen. Die Module sind zur Behandlung und/oder Pflege von Patienten ausgebildet und in einem Behälter angeordnet. Das System umfasst weiter mindestens einen Rechner mit einer Empfangseinheit zur Kommunikation mit mindestens einer Sendeeinheit eines Komponenten-Sets. Bevorzugt findet diese Kommunikation über ein Netzwerk, besonders bevorzugt über ein drahtloses Netzwerk statt. Das System ist ausgebildet, um Änderungen an einem Zustand und/oder Bestand mindestens eines Komponenten-Sets zu erkennen und zu protokollieren.

Obschon im vorliegenden Aspekt die Versorgung eines Gebäudes und/oder Gebäudekomplexes im Vordergrund steht, so eignet sich das erfindungsgemässe System auch zur Versorgung nicht statischer Komplexe. So kann zum Beispiel auch ein Fuhrpark an Fahrzeugen mit dem System gemäss der vorliegenden Erfindung ausgestattet sein. In einem besonderen Beispiel wird ein Fuhrpark an Baustellenfahrzeugen mit dem System zur Versorgung mit Notfallausrüstung zur medizinischen Erstversorgung eines Patienten ausgestattet. Die Vorteile der vorliegenden Erfindung lassen sich auch in einer solchen Umsetzung verwirklichen.

In einer besonderen Ausführungsform ist das System mit einer Datenbank verbunden, welche Informationen zu Bestand und/oder Integrität und/oder Ablaufdatum einzelner Module enthält. Dadurch kann stets gewährleistet werden, dass eine Kontrolle des Bestands und der Integrität der einzelnen Notfallmodule in allen Komponenten-Sets in Echtzeit zur Verfügung steht. Der Rechner kann ausgebildet sein, um eine Bestandesaufnahme auszulösen. Dazu versendet der Rechner z.B. ein Signal an alle angeschlossenen Komponenten-Sets, welche diese veranlassen, eine Bestandesaufnahme durchzuführen. Wie eine Bestandesaufnahme ablaufen kann, wurde oben bereits beschrieben. Die einzelnen Komponenten-Sets melden nun dem Rechner über ihre Sendeeinheiten zurück, wie der Inhalt und die Zusammensetzung ihrer Module zum Zeitpunkt der Bestandesaufnahme bestellt ist. Sollte diese Bestandesaufnahme darauf hinweisen, dass ein Handlungsbedarf besteht, so kann bereits das System veranlassen, dass die entsprechenden Komponenten ersetzt/nachbestellt werden. Dazu kann ein automatisierter Bestellvorgang in die Wege geleitet werden, welche / welcher bei einem Zulieferer die entsprechende Menge an Modulen bestellt, die zu ersetzen sind.

Ein weiterer Aspekt der vorliegenden Erfindung betrifft ein Verfahren zum Betreiben eines Systems zur Versorgung eines Gebäudes und/oder Gebäudekomplexes mit Notfallausrüstung zur medizinischen Erstversorgung eines Patienten. Besonders bevorzugt betrifft dieses Verfahren ein Betreiben eines Systems, wie oben bereits beschrieben. Das Verfahren umfasst ein Bereitstellen mindestens eines Komponenten-Sets zur medizinischen Erstversorgung eines Patienten. Bevorzugt wird ein Komponenten-Set (wie oben beschrieben) bereitgestellt. Das Verfahren umfasst weiter ein Erfassen aller Module, die Bestandteil des/der bereitgestellten Komponenten-Set(s) sind. Diese Module dienen der Behandlung und/oder Pflege eines Patienten und sind entsprechend bestückt. Bevorzugt sind die Module zur Behandlung und/oder Pflege eines Patienten bestückt, der unter einem spezifischen bestimmten Zustand leidet.

In einer besonders bevorzugten Ausführungsform sind die Module jeweils einem bestimmten Zustand zugeordnet. Die Module werden in einer Datenbank erfasst. Das erfindungsgemässe Verfahren umfasst weiter das Empfangen von Statusinformationen des/der Komponenten-Set(s). Das Verfahren umfasst weiter ein Protokollieren der empfangenen Statusinformationen. Als Protokollieren kann im Sinne der vorliegenden Erfindung das Erfassen und Abspeichern von Informationen verstanden werden bspw.

Im Sinne der vorliegenden Erfindung können Statusinformationen Informationen bezüglich des Vorhandenseins eines entsprechenden Moduls im Behälter betreffen. Wie eine solche Bestandesaufnahme durchgeführt werden kann, wurde bereits hinsichtlich des Komponenten-Sets oben erläutert.

In einer besonderen Ausführungsform umfasst das Verfahren einen Schritt, bei dem ein beim Empfangen von Statusinformationen eines Komponenten-Sets als fehlend erkanntes Modul in einem virtuellen Bestellverfahren bestellt wird. Dies kann z.B. dadurch bewerkstelligt werden, dass die empfangenen Statusinformationen mit den Informationen verglichen werden, die in der Datenbank erfasst wurden. Ein möglicher weiterer Schritt wäre es, einen Soll-Wert festzulegen und zu erfassen, der festlegt, wo welche Module vorhanden sein sollten. Bei den Bestandesaufnahmen werden aktuelle Bestände aufgerufen und mit diesem Soll-Wert verglichen. So können festgestellte Abweichungen mit entsprechenden Handlungen korrigiert werden. Ein Beispiel wäre das genannte Bestellverfahren. Alternativ oder ergänzend kann auch ein manuelles Bestücken eines Komponenten-Sets ausgelöst werden, z.B. indem einer verantwortlichen Person eine entsprechende Nachricht zugespielt wird.

In einer besonderen Ausführungsform des erfindungsgemässen Verfahrens umfasst das Erfassen aller Module weiter das Erfassen eines Ablaufdatums jedes Moduls. Das Erfassen aller Module kann weiter das Erfassen von Informationen bezeichnend für ein individuelles Komponenten-Set umfassen. Das Erfassen aller Module kann zudem weiter das Erfassen von Koordinaten eines Komponenten-Sets umfassen. In einer besonderen Ausführungsform kann das Erfassen aller Module das Erfassen von Lagerungsdefinitionen aller erfassten Module umfassen. Solche Lagerungsdefinitionen können zum Beispiel die Umweltparameter umfassen, die für die zuverlässige Lagerung der Module vorherrschen müssen, wie z.B. Temperatur, Feuchtigkeit, Staub, etc. In besonderen Umgebungen können die Ablaufzeiten der Module an die herrschenden Umweltbedingungen angepasst werden und entsprechend protokolliert werden. Mit dem erfindungsgemässen System wird es somit möglich auch innerhalb einer Vielzahl von klimatischen Lagerbedingungen stets Notfallmaterial bereitzustellen, welches den gesetzlichen und betrieblichen Anforderungen entspricht.

Mit diesen Informationen ist es möglich, akkurat und zeitnah Buch zu führen, wo welche Module im Bestand eines Komponenten-Sets vorhanden sind. Allfällige Mängel können somit rasch behoben werden.

In einer besonderen Ausführungsform, wo die Koordinaten eines Komponenten-Sets ermittelt werden (wie oben bereits beschrieben), können diese Koordinaten mit dem Standort, an dem ein Komponenten-Set verwendet wird, verglichen werden. Stellt man fest, dass der Gebrauchsstandort eines Komponenten-Sets von einem Lagerungsstandort eines Komponenten-Sets massgeblich abweicht, so kann man neue Bedürfnisse im System zur Bereitstellung einer medizinischen Erstversorgung eines Patienten in einem Gebäude und/oder Gebäudekomplex ergänzen und überarbeiten. Zudem können die Koordinaten dazu beitragen, ein allfällig nicht wieder verstautes oder gar entwendetes Komponenten-Set aufzufinden und an den angestammten Platz zurückzubefördern.

In einer besonderen Ausführungsform wird beim Öffnen eines verschliessbaren Behälters eines Komponenten-Sets zur medizinischen Erstversorgung eine Steuereinheit hochgefahren, welche eine Sendeeinheit zur Kommunikation mit einem Rechner steuert, sowie ein Sensorelement zur Detektion eines geöffneten oder geschlossenen Zustands steuert. Weiter kann die Steuereinheit eine Bestandesaufnahme steuern, bei der über eine Antenne festgestellt wird, ob und welche Module innerhalb des Behälters aus ihren zugeordneten Bereichen entnommen wurden. Das erfindungsgemässe Verfahren kann weiter dahingehend ergänzt werden, dass die Bestandesaufnahme innerhalb vordefinierter Zeitintervalle stattfindet. Bevorzugt findet die Bestandesaufnahme in Zeitintervallen von einem Monat bis einem Jahr statt, besonders bevorzugt von sechs Monaten statt.

In einer besonderen Ausführungsform des erfindungsgemässen Verfahrens schaltet die Steuereinheit von einem Stand-by-Modus in einen aktiven Modus um, wenn die Stromzufuhr zur Batterie unterbrochen wurde.

In einer weiteren besonderen Ausführungsform wird die Steuereinheit hochgefahren, wenn ein Komponenten-Set nach dem Gebrauch wieder an seinem angestammten Platz verstaut ist. Dies kann bspw. dadurch ausgelöst werden, dass eine entsprechende Befestigungsvorrichtung am Komponenten-Set mit einer Wirkverbindung zur Steuereinheit ausgestattet ist, die wie ein Schalter die Steuereinheit hochfährt, wenn das Komponenten-Set mit einer entsprechenden Vorrichtung verbunden wird. In seiner einfachsten Ausführungsform kann die Befestigungsvorrichtung ein Haken sein, der mit einem entsprechenden Gegenstück an der Wand in eine kraftschlüssige Verbindung eintreten kann.

In einer weiteren besonderen Ausführungsform des erfindungsgemässen Verfahrens werden eine Reihe von Komponenten-Sets regelmässig überprüft, indem Statusinformationen der Komponenten-Sets empfangen werden. Dazu kann z.B. ein Initiationssignal von einem Rechner mittels eines Netzwerks, besonders bevorzugt eines drahtlosen Netzwerks an eine Reihe an Komponenten-Sets versandt werden, wonach diese eine Bestandesaufnahme ihrer Module durchführen. Die Ergebnisse dieser Bestandesaufnahme werden an den Rechner zurückgeschickt, der diese empfängt und protokolliert.

Mit der erfindungsgemässen Lösung wird ein Komponenten-Set, ein System und ein Verfahren bereitgestellt, bei dem eine medizinische Erstversorgung eines Patienten in einem Gebäude und/oder Gebäudekomplex wartungsarm und zuverlässig bereitgestellt werden kann.

Die zahlreichen besonderen Ausführungsformen bieten jeweils eigene zusätzliche vorteilhafte Ausgestaltungen einer möglichen Umsetzung der erfindungsgemässen Lösung.

Im Folgenden soll nun die Erfindung anhand konkreter Ausführungsbeispiele und Figuren näher erläutert werden, ohne jedoch auf diese beschränkt zu sein. Für einen Fachmann ergeben sich aus dem Studium dieser Beispiele allerdings weitere bevorzugte Ausführungsformen, die in einer erfindungsgemässen Lösung umgesetzt werden können.

In den folgenden schematischen Figuren werden der Einfachheit halber die gleichen Bauteile mit den gleichen Bezugszeichen versehen.

### Figurenbeschrieb

Anhand der nachfolgenden Figuren werden Ausführungsbeispiele der Erfindung beschrieben. Es zeigen
Fig. 1 zeigt schematisch eine Ausgestaltung eines erfindungsgemässen Komponenten-Sets;
Fig. 2 zeigt eine alternative Ausgestaltung eines erfindungsgemässen Komponenten-Sets;
Fig. 3 zeigt schematisch ein offenes Komponenten-Set gemäss Erfindung;
Fig. 4 zeigt ein erfindungsgemässes System;
Fig. 5 zeigt schematisch die Anordnung von Modulen in einem erfindungsgemässen Komponenten-Set.

### Ausführung der Erfindung

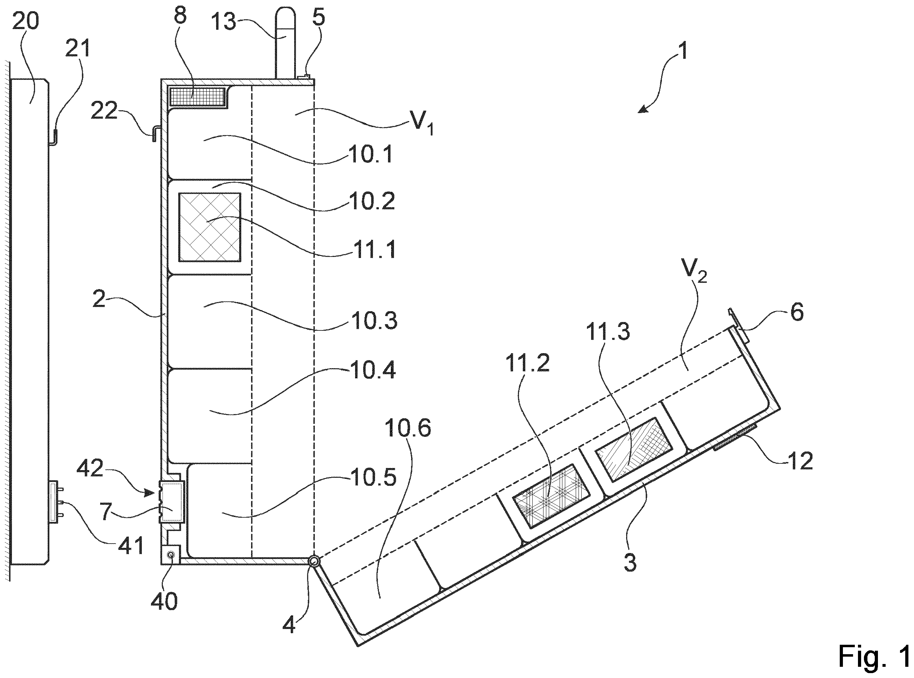

Ein erfindungsgemässes Komponenten-Set 1 ist in der Fig. 1 dargestellt. Das gezeigte Komponenten-Set 1 umfasst zwei Behälterwände 2, 3, welche über ein Scharnier 4 von einem geschlossenen in einen geöffneten (dargestellt) Zustand überführt werden können. Die beiden Behälterwände 2, 3 bilden gemeinsam einen verschliessbaren Behälter. Eine erste Behälterwand 2 ist insgesamt 350 mm hoch (in der Bildebene die y-Achse) und 10 mm breit (die x-Achse). Die z-Achse, d.h. die Ausdehnung der ersten Behälterwand 2 in die Betrachterebene, beträgt 250 mm. Diese Angaben sind rein exemplarisch für dieses konkrete Ausführungsbeispiel. Die erste Behälterwand ist einstückig aus einem spritzgegossenen Kunststoffkörper ausgebildet. Im Innern wird ein erstes Innenvolumen V1 von der ersten Behälterwand 2 umschlossen. Dieses erste Volumen V1 ist weiter unterteilt in einer Reihe an Bereichen 10.1, 10.2, 10.3, 10.4, 10.5. Die Bereiche können in ihren Ausmassen unterschiedlich ausgestattet sein, je nach Modulen 11.1, die sie beherbergen sollen. Das Komponenten-Set 1 ist so ausgebildet, dass eine Reihe an Modulen 11.1, 11.2, 11.3 im Komponenten-Set 1 angeordnet werden können. Die Module 11.1, 11.2, 11.3 sind entnehmbar und dienen der Behandlung eines bestimmten Zustands, insbesondere eines bestimmten Notfalls. Im vorliegenden Beispiel sind lediglich drei Volumen aus Illustrationsgründen dargestellt. Das gezeigte Komponenten-Set 1 ist aber ausgelegt, um eine Mehrzahl an solchen Modulen aufzunehmen. In einem konkreten Beispiel können 22 Module aufgenommen werden. Dabei sind gewisse Modultypen, die für bestimmte Behandlungen eingesetzt werden sollen, in mehrfacher Ausführung vorhanden. Bestimmte, eher seltener verwendete Modultypen können in einfacher Ausführung vorkommen.

Das als Beispiel gezeigte Komponenten-Set umfasst Module mit konfektionierten Zusammensetzungen aus Wundverbänden, Druckverbänden, Kompressen zur Wundversorgung, Kaltkompressen, Warmkompressen, eine Hyperventilationsmaske, ein Satz Splitterpinzette und Skalpell, eine Rettungsdecke, fünf abgepackte sterile Handschuhe, Desinfektionsmittel und ein Set an Holzspachteln.

Das Verbandsmaterial kann in mehreren Modulen wiederholt im Komponenten-Set vorkommen. So kann z.B. ein Komponenten-Set zur Behandlung von Quetschungen ein Dreieckstuch aufweisen, während ein Modul zur Behandlung von Kopfverletzungen ebenfalls ein Dreieckstuch aufweisen kann.

Die Zusammensetzung der Module und die Zusammensetzung des konfektionierten Modulinhalts können auf individuelle Kundenwünsche angepasst werden. So ist es z.B. möglich, anhand des Betriebs festzustellen, welche Module besonders sinnvoll sind. Ebenfalls ist es möglich, innerhalb eines Betriebs verschiedene Bereiche mit unterschiedlich ausgestatteten Komponenten-Sets zu versorgen. Ein Vorteil der erfindungsgemässen Lösung ist es eben, dass trotz unterschiedlicher Konfiguration der Komponenten-Sets in einem System stets Übersicht besteht, wo welche Module bereitgestellt werden.

Das Komponenten-Set 1 weist einen definierten ersten Bereich 10.2 auf, der für die Aufnahme eines bestimmten Moduls 11.1 ausgelegt ist. Um unmissverständlich klarzumachen, dass dieser Bereich zur Verstauung dieses bestimmten Moduls 11.1 zu verwenden ist, kann dieser erste Bereich 10.2 mit einer entsprechenden farblichen Kennzeichnung versehen sein. Dieser farblichen Kennzeichnung können farblichen Kennzeichnungen auf einer Modulverpackung des ersten Moduls 11.1 entsprechen. Sind mehrere Bereiche 10.2, 10.3, 10.4 so ausgebildet, dass sie einen bestimmten Modultyp 11.1 aufnehmen können, so können diese Bereiche 10.2, 10.3, 10.4 neben ihrer identischen Abmessung zusätzlich auch die gleiche Kennzeichnung aufweisen. Die Zuordnung eines Moduls zu einem bestimmten Bereich kann selbstverständlich auch anhand der Form als solche von vornherein klar sein. Für weitere Modultypen (nicht gezeigt) können in der ersten Behälterwand 2 entsprechend angepasste Bereiche 10.1, 10.5 vorgesehen sein. In einem zweiten Bereich 10.1 kann z.B. die Aufnahme einer Rettungsdecke vorgesehen sein. In einem dritten Bereich 10.5 können z.B. Desinfektionsmittel und sterile Handschuhe in einem Modul untergebracht werden.

In einer besonders bevorzugten Ausführungsform wird das gesamte Innenvolumen V1 der ersten Behälterwand 2 für relevante Module verwendet. Die erste Behälterwand 2 in diesem Beispiel weist auch am Formkörper ausgenommene Bereiche auf, die weitere Elemente beherbergen. So weist die erste Behälterwand 2 ein zum Innenvolumen V1 abgetrenntes Kontrollabteil auf, das eine Kontrolleinheit 8 beherbergt. Die Kontrolleinheit 8 umfasst wiederum eine Sendeeinheit zur Kommunikation mit einem Rechner und eine Steuereinheit zur Verarbeitung von Messwerten und Signalen. Zum Empfang von Messwerten und Signalen erstreckt sich vom Kontrollabteil 8 ein Geflecht an Antennen, welche die gesamte Rückwand des Komponenten-Sets 1 durchziehen und dergestalt an den Bereichen 10.1, 10.2, 10.3, 10.4, 10.5 vorbeigeführt werden, dass ein in diesen Bereichen emittiertes elektromagnetisches Signal empfangen werden kann. Diese Antennen sind in der Fig. 1 nicht explizit dargestellt. Zudem erstreckt sich vom Kontrollelement 8 eine Stromverbindung zu einem Netzteil 7, welches wiederum eine Steckverbindung aufweist, die mit einem Stromstecker 41 verbunden werden kann. Diese Steckverbindung Z kann so ausgestaltet sein, dass sie von aussen relativ zum Innenvolumen des Behälters zugänglich ist.

Im vorliegenden Beispiel weist das Verschlusssystem des Komponenten-Sets einen Reed-Schalter auf, der mittels hermetisch verkapselter Kontaktzungen berührungslos mittels eines Magnetfeldes ein Öffnen und/oder Schliessen des Behälters zu detektieren vermag.

In einer alternativen Ausführung des Komponenten-Sets 1 kann anstelle des Antennengeflechts eine einzelne Antenne vorgesehen sein, welche bei der Entnahme einzelner Module das entnommene Modul ausliest. Dies kann z.B. vonstattengehen, indem die Antenne in einem separaten Lesebereich des Komponenten-Sets 1 angebracht ist, an dem die entnommenen Module vorbeigeführt werden. Die Steuereinheit registriert die von der Antenne empfangene Kennung bezüglich der Module und verfährt mit dieser Information wie mit einer Kenntnis, die aus dem Antennennetz erlangt worden wäre.

Im vorliegenden Beispiel umfasst die Kontrolleinheit einen Mikrocontroller mit einem Prozessor, sowie einem Arbeitsspeicher und einem Programmspeicher. Der Mikrocontroller ist ausgelegt, um Sensorinformationen von verschiedenen Elementen des Komponenten-Sets zu erhalten und zu verarbeiten. Unter anderem steht er mit einer Sendeeinheit in Wirkverbindung. Im vorliegenden Beispiel ist die Sendeeinheit ein integriertes Modem. In diesem konkreten Beispiel wurde ein LoRa-Modem verwendet mit einer Reichweite von bis zu 15 km. Diese Module verfügen über einen geringen Stromverbrauch und eine lange Laufzeit. Weiter ist die Kontrolleinheit 8 mit einer Speisung verbunden (im vorliegenden Beispiel einem Netzteil 7). Ein weiterer NFC-Sensor steht ebenfalls mit der MCU in Wirkverbindung und dient der Erkennung der Identifikationsmittel der Module.

Im vorliegenden Beispiel verfügen alle Module 11.1, 11.2, 11.3 über eigene Identifikationsmittel. Dies kann im vorliegenden Beispiel durch RFID-Tags passiver Art gewährleistet werden, welche entsprechende Informationen enthalten bezüglich des Herstellungsdatums, des Inhalts und des Ablaufdatums der konfektionierten Bestandteile des entsprechenden Moduls. Im Betrieb, wenn die Identifikationsmittel ausgelesen werden, verarbeitet die Kontrolleinheit diese Informationen und übermittelt sie mittels der integrierten Sendeeinheit an einen externen Rechner (in der Fig. 1 nicht dargestellt). Die erste Behälterwand ist zur Befestigung an eine Wandkonsole 20 konzipiert. Zur Befestigung an die Wandkonsole 20 verfügt die erste Behälterwand 2 über einen Befestigungshaken 22. Wahlweise kann dieser Befestigungshaken 22 ein elektrisches Signal auslösen, welches von der Kontrolleinheit 8 erfasst und verarbeitet wird. Dieses elektrische Signal kann Informationen hinsichtlich der Positionierung der ersten Behälterwand an der Wandkonsole 20 enthalten. So kann z.B. eine Entnahme des Komponenten-Sets 1 von der Wandkonsole 20 detektiert werden und bestimmte Prozesse auslösen.

Selbstredend verfügt die Wandkonsole 20 über ein entsprechendes Hakengegenstück 21, an dem der Wandhaken 22 der ersten Behälterwand 2 in kraftschlüssiger Verbindung montiert werden kann. Im gezeigten Beispiel ist zudem die Stromzufuhr 41 so ausgelegt, dass sie bei korrekt montiertem Komponenten-Set mit der Steckverbindung 42 in Wirkverbindung tritt, sodass eine Batterie des Komponenten-Sets 1 geladen wird.

Im vorliegenden Beispiel verfügt die erste Behälterwand 2 zudem über einen abgegrenzten Bereich zur Aufnahme eines GPS-Moduls 40. Dieses GPS-Modul 40 kann z.B. mit einer SIM-Karte ausgestattet werden, womit das Komponenten-Set 1 stets zuverlässig anhand der Koordinaten geortet werden kann. Das GPS-Modul 40 kann mit einem GSM-Sender ausgestattet werden und über eine eigene Energiespeisung verfügen oder mit der Batterie und dem Netzteil 7 verbunden sein.

Die erste Behälterwand 2 ist über eine Scharnier 4 mit einer zweiten Behälterwand 3 verbunden. Diese zweite Behälterwand 3 umfasst ebenfalls ein zweites Innenvolumen V2, welches sich wiederum in eine Reihe von Bereichen 10.6 unterteilt, welche zur Aufnahme von Modulen ausgebildet sind. In der Fig. 1 sind exemplarisch lediglich zwei Module 11.2, 11.3 abgebildet. Diese zweite Behälterwand ist mit einem Verschlusselement 6 ausgestattet, welches mit einer Rastnut 5 der ersten Behälterwand 2 in Wirkverbindung treten kann, sodass der Behälter im geschlossenen Zustand gesichert ist. Neben dieser einfachen Rastverbindung sind zahlreiche andere Mittel zum Verschliessen des Koffers geeignet. Im vorliegenden Beispiel ist zudem über diese Verbindung detektierbar, ob ein Koffer geöffnet werden kann. Ebenfalls denkbar ist ein einfacher Lichtsensor, der feststellt, ob ein Koffer geöffnet wurde und entsprechend diese Öffnung von der Kontrolleinheit 8 zur Verarbeitung übermittelt.

Das Komponenten-Set verfügt für den einfacheren Transport über einen Tragegriff 13. Der Tragegriff 13 ist bevorzugt so positioniert, dass er im Wesentlichen dem Schwerpunkt des Komponenten-Sets in seiner Querausdehnung entspricht, was ein Tragen erleichtert. Die Ausmasse der zweiten Behälterwand 3, welche im vorliegenden Fall eine komplementäre Schalenhälfte zu einem als Koffer ausgebildeten Komponenten-Set 1 mit der ersten Behälterwand 2 ein gemeinsames Innenvolumen V1 und V2 bildet, entspricht in der Höhe und der Breite den Ausmassen der ersten Behälterwand 2. Die Dicke der zweiten Behälterwand ist im vorliegenden Beispiel etwas geringer ausgebildet und umfasst 80 mm.

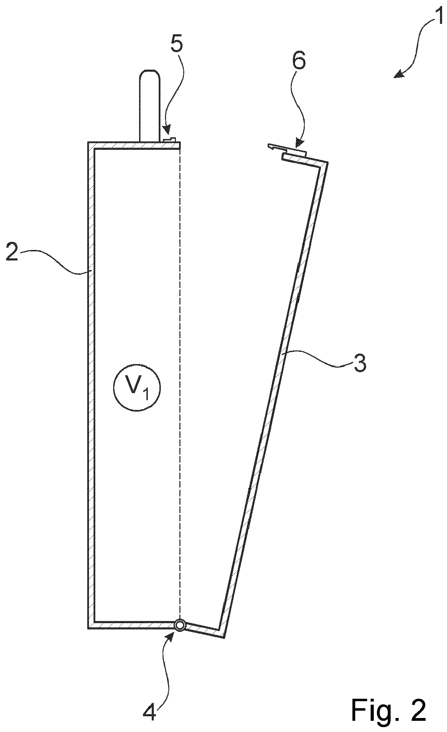

Viele der dargestellten Funktionen sind fakultative Ausführungen einer erfindungsgemässen Lösung. In seiner einfachsten Ausführungsform umfasst das Komponenten-Set lediglich das Innenvolumen, die Bereiche, sowie die Kontrolleinheit 8 und eine Speisung und ein Mittel zur Detektion einer Öffnung des Komponenten-Sets. Geeignete Mittel wurden vorgängig beschrieben. Im Betrieb würde in dieser einfachsten Lösung eine Entnahme des Komponenten-Sets von der Wandkonsole 20 noch kein Signal auslösen. Wird das Komponenten-Set allerdings geöffnet, so würde die Kontrolleinheit ihre Arbeit aufnehmen und der Mikrocontroller die Öffnung über die Sendeeinheit an einen externen Rechner vermelden. Bereits eine solche Ausführung bedeutet in diesem technischen Gebiet eine erhöhte Sicherheit, da geöffnete Komponenten-Sets für die Kontrolle vorgesehen werden können. Entsprechend ausgebildetes Personal kann dann die Integrität und den Bestand der Module des Komponenten-Sets überprüfen. Die in der Fig. 2 gezeigte alternative Ausführungsform umfasst lediglich die erste Behälterwand 2 mit einem ersten Innenvolumen V1 und eine zweite Behälterwand 3, die lediglich als Deckel für die erste Behälterwand 2 ausgebildet ist. Dieses Komponenten-Set 1 unterteilt das erste Innenvolumen V1 selbstverständlich erneut in entsprechende Bereiche für die Module zur Behandlung spezifischer Zustände. Im Unterschied zum vorangegangenen Beispiel des Komponenten-Sets 1 wird allerdings die zweite Behälterwand 3 auf ihre Funktion als Deckel beschränkt. Diese Komponenten-Sets 1 können durch die bessere Tragbarkeit in Bereichen, wo eine erhöhte Mobilität wichtig ist, eingesetzt werden.

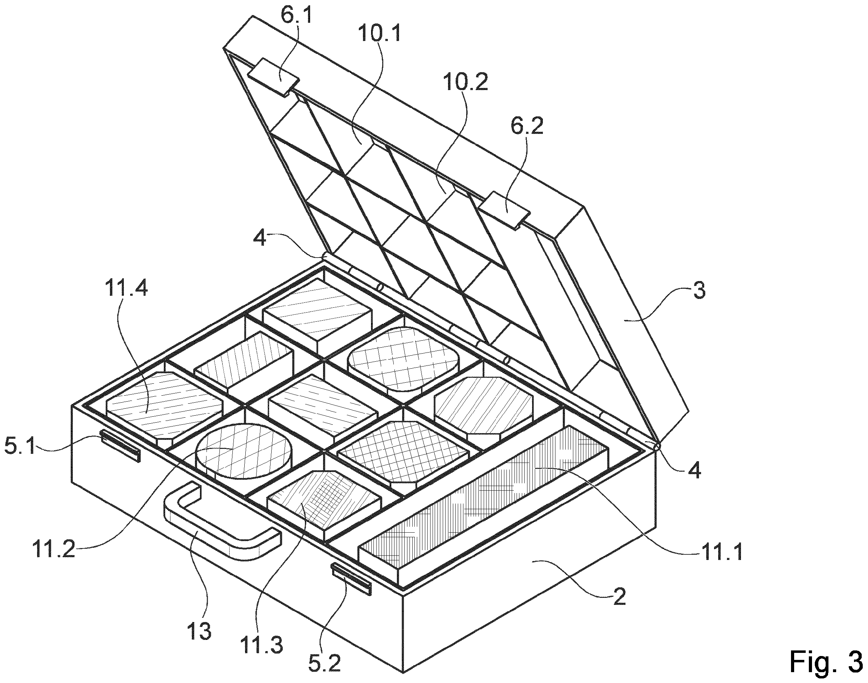

In der Fig. 3 ist nun schematisch eine Anordnung verschiedener Module 11.1, 11.2, 11.3, 11.4 in einem Komponenten-Set 1 dargestellt. Das Komponenten-Set 1 besteht aus zwei Behälterwänden 2, 3, welche als Koffer mit einer Scharnier 4 miteinander als Kofferhälften verbunden sind. Die erste Behälterwand 2 weist zudem einen Tragegriff 13 auf, sowie zwei Rastnasen 5.1, 5.2, welche mit den entsprechenden Verschlüssen 6.1, 6.2 der zweiten Behälterwand 3 verrasten. Das Innere der ersten Behälterwand ist unterteilt in Bereiche verschiedener Grösse. Jedes der dargestellten Module 11.2, 11.3, 11.4, 11.1 dient einer bestimmten Behandlung eines definierten pathologischen Zustands oder Unfalls. So kann das Modul 11.1 eine zusammengerollte Löschdecke umfassen, welche im Brandfall anzuwenden wäre. Das Modul 11.2 kann entsprechende Kühlkompressen mit einem Gel umfassen. Das Modul 11.3 kann verschiedene Pflaster und Verbandsmaterialien zur Verpflegung von Schnitt- und Stichwunden umfassen, während das Modul 11.4 für Quetschungen, Dreieckstücher, Verbandsmaterial und Kühlsprays umfasst. Entsprechend können auch Bereiche in der zweiten Behälterwand 3 vorgesehen sein 10.1, 10.2, welche weitere Module zeigen. Die Module sind hier exemplarisch anhand ihrer Schraffierung individuell dargestellt. Neben der Form und der Schraffierung können die Module auch mit Piktogrammen versehen sein, welche die entsprechende Behandlung symbolisch darstellen.

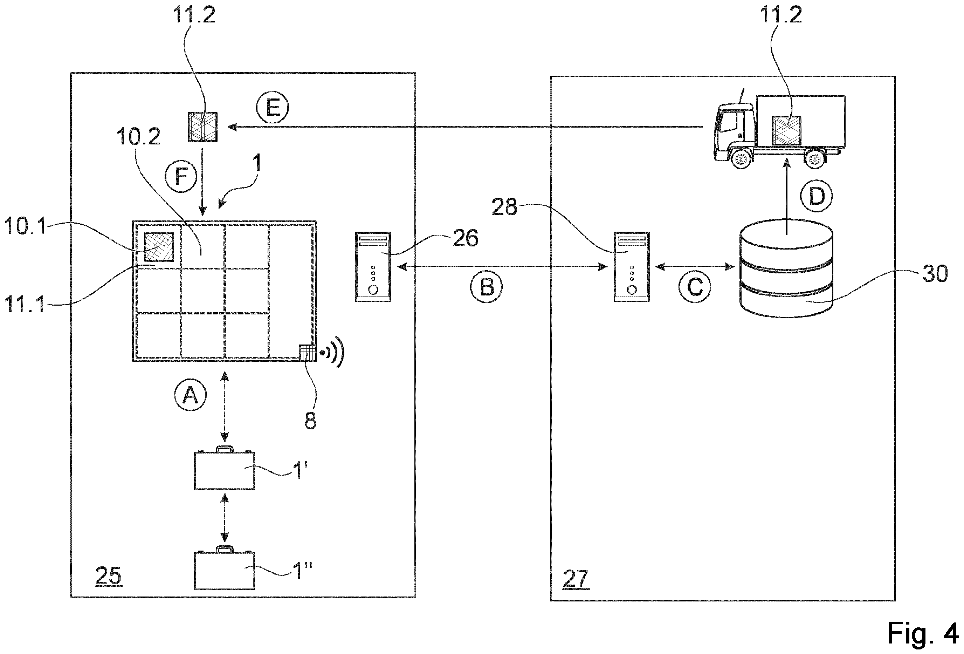

Die Fig. 4 zeigt die Umsetzung der vorliegenden Erfindung als System zur Sicherstellung einer medizinischen Erstversorgung in einem Gebäudekomplex. Der Gebäudekomplex ist hier schematisch als erstes Umfeld 25 dargestellt. In diesem ersten Umfeld 25 sind eine Reihe an / von Komponenten-Sets 1, 1', 1" an zugänglichen Orten angebracht. Die Komponenten-Sets 1, 1', 1" verfügen über eine Kontrolleinheit, welche in der Lage ist, über ein drahtloses Netzwerk mit einem betriebsinternen Rechner 26 zu kommunizieren. Tritt nun ein Notfall auf und ein erstes Komponenten-Set 1 wird in Betrieb genommen, so erwacht die Kontrolleinheit 8 zum Leben. Wird im Betrieb ein entsprechendes Modul entnommen, so registriert in einem ersten Schritt A die Kontrolleinheit das Fehlen eines Moduls in einem bestimmten Bereich 10.2. In einem anderen Bereich 11.1 wird von der Kontrolleinheit das Vorhandensein des entsprechenden Moduls 10.1 ebenfalls registriert. Diese Informationen werden anhand von Identifikationsmitteln an den Modulen ermittelt und von der Kontrolleinheit an einen Rechner 26 übermittelt. Dieser Rechner 26 nimmt ein Kommunikationsprotokoll B auf, mit einem weiteren Rechner 28 eines Zulieferers, welcher hier beispielhaft als Zuliefererumgebung 27 dargestellt wird. Dieser Rechner 28 vergleicht nun die erhaltenen Ist-Werte bezüglich der Komponenten-Sets 1, 1', 1" der Umgebung 25 mit einem protokollierten Soll-Zustand C. Werden Abweichungen zum Soll-Zustand C festgestellt, indem z.B. ein in einer Datenbank 30 abgespeicherter Soll-Zustand auf Module hinweist, die im entsprechenden Umfeld 25 in einzelnen Komponenten-Sets vorhanden sein sollten, so wird ein Bestellvorgang D ausgelöst, welcher ein entsprechendes Modul 10.2 umfasst, welches als Fehlen erkannt wurde. Dieses Modul 10.2 wird an die Umgebung 25 geliefert E und mit entsprechenden Anweisungen versehen, sodass ein entsprechende Bestückung F des Komponenten-Sets 1 stattfinden kann. Mit den entsprechenden Angaben kann eine verantwortliche Person das Komponenten-Set 1 ausfindig machen und das fehlende Modul 10.2 in den Bereich 10.2 überführen F.

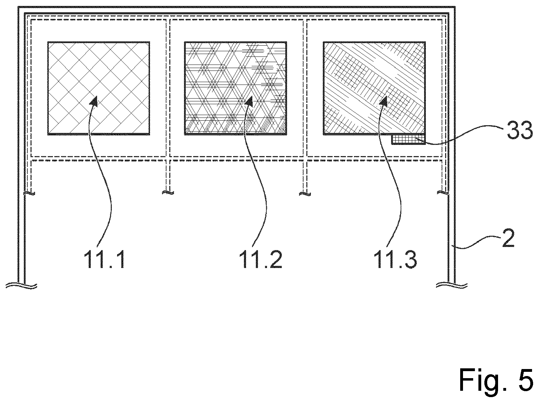

Fig. 5 zeigt die Zuordnung einzelner Module 11.1, 11.2, 11.3 in einer ausschnittsweise gezeigten Behälterwand 2. Die gestrichelte Linie zeigt einen Einsatz, im vorliegenden Fall ein Schaumstoffgitter, in dem die einzelnen Module angebracht werden können. Das Modul 11.3 zeigt zudem exemplarisch einen besonderen Transponder, der mit dem Modul 11.3 so verbunden ist, dass der Transponder beim Öffnen des Moduls unwiderruflich beschädigt wird. Ein solches Modul 11.3 würde selbst dann als fehlend vom Komponenten-Set angesehen werden, wenn es zurück in das Komponenten-Set an die entsprechend richtige Bereich versorgt würde.

Mit der vorliegenden Erfindung ist nun ein Komponenten-Set, ein System und ein entsprechendes Verfahren bereitgestellt, welches die Kontrolle und die Aufrechterhaltung einer Versorgung durch medizinisches Notfall- und Pflegematerial in einem Gebäude oder / und/oder Gebäudekomplex vereinfacht und sicherer macht.

## Patentansprüche

1. Komponenten-Set (1) zur medizinischen Erstversorgung eines Patienten umfassend:
a. einen verschliessbaren Behälter mit einer ersten Behälterwand (2) die mindestens ein erstes Innenvolumen (V1) definiert;
b. eine Mehrzahl an zur Anordnung im ersten Innenvolumen vorgesehenen Modulen (11.1, 11.2, ...), wobei die Module (11.1, 11.2, ...) zur Behandlung und/oder Pflege eines Patienten ausgebildet sind, insbesondere wobei die Module (11.1, 11.2, ...) zur Behandlung und/oder Pflege eines Patienten der unter einem spezifischen Zustand leidet ausgebildet sind;
c. ein Sensorelement zur Detektion eines geöffneten und/oder geschlossenen Zustandes des verschliessbaren Behälters;
d. eine Sendeeinheit zur Kommunikation mit einem Rechner, und
e. eine Steuereinheit zur Verarbeitung der Detektion des Zustands des Behälters, und zur Erzeugung eines Signals.

2. Komponenten-Set gemäss Anspruch 1, wobei die erste Behälterwand (2) definierte Aufnahmebereiche (10.1, 10.2, ...) zur Aufnahme mindestens eines Moduls (11.1, 11.2, ...) der Mehrzahl an Modulen (11.1, 11.2, ...) aufweist, insbesondere zur Aufnahme je eines definierten Typs eines Moduls (11.1, 11.2, ...) der Mehrzahl an Modulen (11.1, 11.2, ...).

3. Komponenten-Set gemäss einem der Ansprüche 1 oder 2, weiter umfassend ein zweites Sensorelement zur Detektion einer Abwesenheit eines bestimmten Moduls (11.1, 11.2, ...) aus der Mehrzahl an zur Anordnung im ersten Innenvolumen ausgebildeten Modulen (11.1, 11.2, ...), insbesondere wobei das zweite Sensorelement zusätzlich zur Detektion einer Abwesenheit eines bestimmten Moduls (11.1, 11.2, ...) aus der Mehrzahl an zur Anordnung im ersten Innenvolumen ausgebildeten Modulen (11.1, 11.2, ...) ausgebildet ist.

4. Komponenten-Set gemäss einem der Ansprüche 1 bis 3, wobei das Komponentenset eine Empfangseinheit zum Empfang von Daten aufweist.

5. Komponenten-Set gemäss einem der Ansprüche 1 bis 4, wobei das Komponentenset eine Quelle (7) für elektrische Energie aufweist.

6. Komponenten-Set gemäss einem der Ansprüche 1 bis 5, umfassend mindestens ein Identifikationsmittel zur Identifikation eines Moduls (11.1, 11.2, ...) der Mehrzahl an Modulen, insbesondere wobei jedes Modul (11.1, 11.2, ...) der Mehrzahl an Modulen (11.1, 11.2, ...) ein eigenes Identifikationsmittel aufweist.

7. Komponenten-Set gemäss einem der Ansprüche 1 bis 6, wobei der Behälter eine zweite Behälterwand (3) aufweist, die mindestens ein zweites Innenvolumen (V2) definiert.

8. Komponenten-Set gemäss einem der Ansprüche 1 bis 7, wobei die erste Behälterwand (2) und/oder die zweite Behälterwand (3) mindestens eine Antenne aufweist/aufweisen, so dass ein elektromagnetisches Signal zur Detektion eines Moduls in ihrem Wirkbereich empfangbar ist, insbesondere ist die Antenne so ausgestaltet, dass ein elektromagnetisches Signal zur Detektion eines Moduls in ihrem Wirkbereich in ihrem Nahfeld empfangbar ist.

9. Komponenten-Set gemäss einem der Ansprüche 1 bis 8, wobei das Sensorelement, die Sendeeinheit und die Steuereinheit in einem definierten Kontrollabteil, das ausgebildet ist im Innenvolumen (V1, V2) der ersten Behälterwand (2) und/oder der zweiten Behälterwand (3), untergebracht sind und so eine Kontrolleinheit (8) bilden, insbesondere wobei das Kontrollabteil gegenüber den definierte Aufnahmebereichen (10.1, 10.2, ...) und dem übrigen Volumen (V1, V2) abgeschlossen ist.

10. System zur Versorgung eines Gebäudes und/oder Gebäudekomplexes mit Notfallausrüstung zur medizinischen Erstversorgung eines Patienten umfassend:
a. eine Mehrzahl an Komponenten-Sets, insbesondere Komponenten-Sets gemäss einem der Ansprüche 1 bis 9, zur medizinischen Erstversorgung eines Patienten, jedes Komponentenset umfassend eine Mehrzahl an Modulen (11.1, 11.2, ...), wobei die Module (11.1, 11.2, ...) zur Behandlung und/oder Pflege eines Patienten ausgebildet sind, insbesondere wobei die Module (11.1, 11.2, ...) zur Behandlung und/oder Pflege eines Patienten der unter einem spezifischen Zustand leidet ausgebildet sind, und wobei besagte Module in einem Behälter angeordnet sind;
b. ein Rechner mit einer Empfangseinheit zur Kommunikation mit mindestens einer Sendeeinheit eines Komponentensets, und wobei
das System ausgebildet ist, um Änderungen an einem Zustand und/oder Bestand mindestens eines Komponenten-Sets zu protokollieren.

11. System gemäss Anspruch 10, wobei das System mit einer Datenbank verbunden ist, die Informationen zu Bestand und/oder Integrität und/oder Ablaufdatum und/oder Lagerungsdefinitionen einzelner Module (11.1, 11.2, ...) umfasst.

12. Verfahren zum Betreiben eins Systems zur Versorgung eines Gebäudes und/oder Gebäudekomplexes mit Notfallausrüstung zur medizinischen Erstversorgung eines Patienten, insbesondere zum Betreiben eines Systems gemäss einem der Ansprüche 10 bis 11, umfassend die Schritte:
a. bereitstellen mindestens eines Komponenten-Sets (1) zur medizinischen Erstversorgung eines Patienten, insbesondere eines Komponenten-Sets gemäss einem der Ansprüche 1 bis 9;
b. erfassen aller Module (11.1, 11.2, ...), die Bestandteil des/der Komponenten-Sets sind, und zur Behandlung und/oder Pflege eines Patienten ausgebildet sind, insbesondere wobei die Module (11.1, 11.2, ...) zur Behandlung und/oder Pflege eines Patienten der unter einem spezifischen Zustand leidet ausgebildet sind, in einer Datenbank;
c. empfangen von Statusinformationen des/der Komponenten-Sets;
d. protokollieren der empfangenen Statusinformationen.

13. Verfahren gemäss Anspruch 12, weiter umfassend den Schritt, dass ein beim Empfangen von Statusinformationen eines Komponenten-Sets als fehlend erkanntes Modul in einem virtuellen Bestellverfahren bestellt wird.

14. Verfahren nach einem der Ansprüche 12 oder 13, wobei das erfassen aller Module unter Schritt b) weiter umfasst das Ablaufdatum jedes Moduls zu erfassen, und/oder Informationen bezeichnend für ein individuelles Komponenten-Set und/oder für die Koordinaten eines Komponentensets zu erfassen.

15. Verfahren gemäss einem der Ansprüche 12 bis 14, wobei die Komponenten-Sets einen verschliessbareren Behälter und ein Sensorelement zur Detektion eines geöffneten Zustands umfassen, und das Verfahren als weitere Schritte umfasst:
a. detektieren eines geöffneten Zustandes des verschliessbaren Behälters, und
b. aktivieren einer Sendeeinheit des betreffenden Komponenten-Sets zur Kommunikation zum Versenden von Statusinformationen.
